(19) Europäisches Patentamt
European Patent Office
Office européen des brevets

(11) Numéro de publication: **0 068 979**
**B1**

(12) **FASCICULE DE BREVET EUROPEEN**

(45) Date de publication du fascicule du brevet: **25.09.85**

(51) Int. Cl.⁴: **C 07 D 333/20**, C 07 D 409/12, C 07 D 333/32 // C07D333/22, C07F9/40

(21) Numéro de dépôt: **82401096.1**

(22) Date de dépôt: **16.06.82**

(54) **Procédé de préparation de dérivés des (thiényl-2)- et (thiényl-3)-2 éthylamines et produits ainsi obtenus.**

(30) Priorité: **30.06.81 FR 8113063**

(43) Date de publication de la demande: **05.01.83 Bulletin 83/1**

(45) Mention de la délivrance du brevet: **25.09.85 Bulletin 85/39**

(84) Etats contractants désignés: **AT BE CH DE FR GB IT LI LU NL SE**

(56) Documents cités:
**CA - A - 1 088 539**
**FR - A - 2 300 090**

**JUSTUS LIEBIGS: Annalen der Chemie, vol. 686, 1965, pages 107-114, Verlag Chemie GmbH, Weinheim, DE. H. ZIMMER et al.: "Synthesen mit Alpha-Amino-Alkyl-Phosphonsäureestern, 1"**
**TETRAHEDRON LETTERS, no. 26, 1979, pages 2433-2436, Pergamon Press Ltd., G.B. N.L.J.M. BROEKHOF et al.: "Enamine synthesis by the horner-wittig reaction"**

(73) Titulaire: **SANOFI, Société dite:, 40, Avenue George V, F-75008 Paris (FR)**

(72) Inventeur: **Chekroun, Isaac, 26 Rue Peyras, F-31000 Toulouse (FR)**
Inventeur: **Heymes, Alain, Chemin de l'Adrech, F-04200 Sisteron (FR)**

(74) Mandataire: **Bressand, Georges et al, c/o CABINET LAVOIX 2 Place d'Estienne d'Orves, F-75441 Paris Cedex 09 (FR)**

**EP 0 068 979 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

**Description**

La présente invention est relative à un nouveau procédé de préparation de thiénylamines répondant à la formule (I)

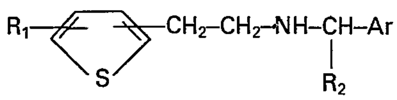 (I)

dans laquelle $R_1$ (en position 2, 3, 4 ou 5) représente un atome d'hydrogène, un radical alkyle ou aldoxy linéaire ou ramifié, un atome d'halogène, un radical nitro, amino.

Dans la chaîne aminoéthyle qui peut occuper les positions 2 ou 3 du noyau thiophénique, $R_2$ signifie un atome d'hydrogène ou un radical alkyle linéaire ou ramifié;

Ar représente un radical thiényle, furyle, pyridyle, phényle ou naphtyle, ou phényle mono- ou poly-substitué par des groupes tels qu'alkyle, phényle, halogène, nitro, cyano, amino carboxy ou alcoxy.

Un certain nombre de dérivés répondant à la formule I sont connus et utilisés comme intermédiaires dans la préparation de composés employés aussi bien dans l'industrie chimique que pharmaceutique.

C'est ainsi, qu'à titre d'exemple, on peut mentionner parmi les dérivés obtenus selon le nouveau procédé de préparation ceux qui peuvent conduire par des moyens connus d'une part (la chaîne aminoéthyle étant en 2 et le radical $R_1$ en 4 ou 5) aux dérivés de la tétrahydro 4, 5, 6, 7 thiéno (3,2-c) pyridine d'autre part (la chaîne aminoéthyle étant en 3 et le radical $R_1$ en 4 ou 5) à ceux de la tétrahydro 4, 5, 6,7 thiéno (2,3-c) pyridine, dérivés qui, dans les deux cas, ont fait l'objet de la part de la présente demanderesse pour leurs applications en thérapeutique et/ou leurs procédés de préparation de plusieurs brevets (Fr. 7303503; Fr. 7503968; Fr. 7520241; Fr. 7523786; Fr. 7524486; Fr. 7600003; Fr. 7721517; CA-A.1088539).

L'invention a pour objet un procédé simple et peu coûteux, en regard de la technique antérieure, d'obtention des composés de formule I.

Conformément au procédé de l'invention, pour préparer les dérivés de formule I:

a) on condense un dérivé de formule II

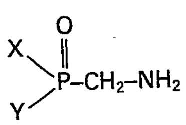 (II)

dans laquelle X et Y sont identiques ou différents et représentent un radical alkyle, aryle, alkoxy, aryloxy, dialkyl – ou diarylamino de telle sorte que le composé organophosphoré de formule II peut être par exemple un phosphonate, un phosphinate, un oxyde de phosphine ou un phosphonamide avec un composé carbonylé de formule III

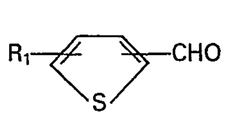 (III)

dans laquelle $R_1$ est tel que défini pour la formule I pour conduire à un composé de formule IV

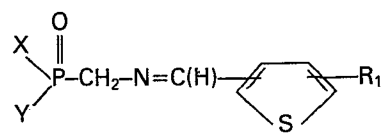 (IV)

dans laquelle les différents radicaux ont la signification fournie précédemment.

b) le composé de formule IV est traité avec une base de formule $B^{\ominus} M^{\oplus}$ pour conduire à un carbanion de formule V

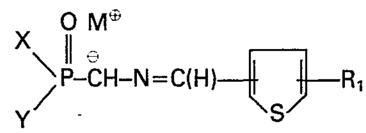 (V)

c) qui se transforme en dérivé VI sous l'action de la chaleur

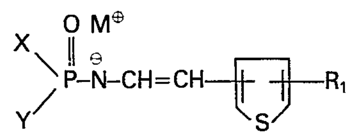 (VI)

pour aboutir, après reprise par l'eau, au composé de formule VII

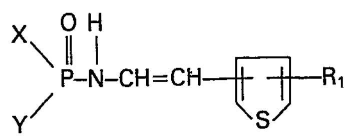 (VII)

Cette réaction est conduite généralement à une température comprise entre −78°C et +150°C, choisie plus spécifiquement en fonction de la base $B^{\ominus} M^{\oplus}$ dans une plage plutôt haut de gamme, en particulier lors de la réalisation de la phase c.

d) le composé de formule VII est mis à réagir successivement avec une base de formule $B'^{\ominus} M'^{\oplus}$ puis un composé carbonylé de formule VIII

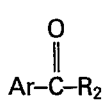 (VIII)

dans laquelle Ar et $R_2$ sont tels que définis pour la formule I, pour obtenir un composé de formule IX

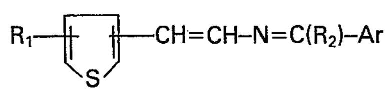 (IX)

e) le dérivé de formule IX est finalement transformé sous l'action d'un agent réducteur tel que notamment un borohydrure de métal alcalin en composé de formule I tel que défini plus haut.

Le procédé de préparation suivant l'invention peut être illustré par le schéma réactionnel suivant:

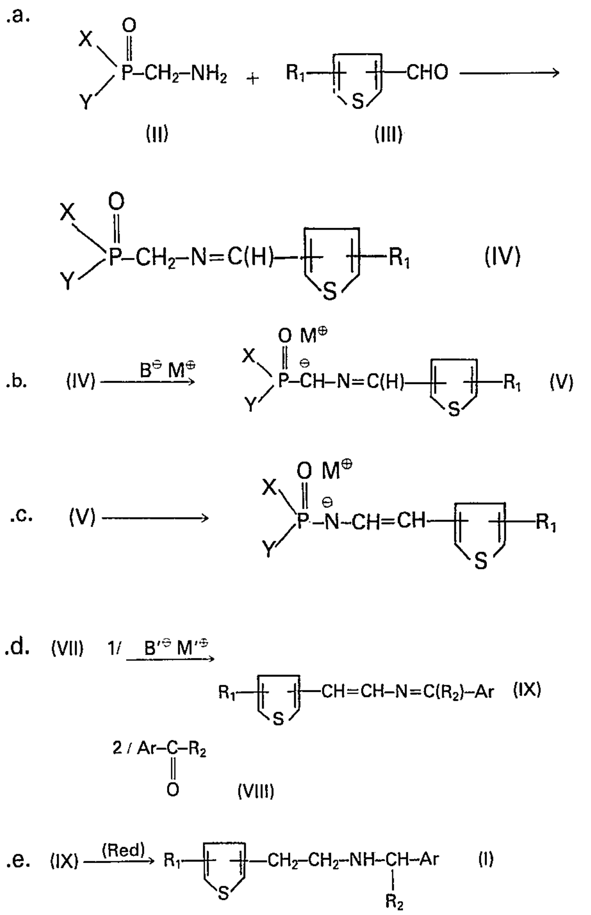


Le procédé peut avantageusement être mis en œuvre comme suit:

a) les composés organophosphorés de formule II, facilement accessibles par des procédés d'obtention bien connus tel que celui, par exemple, décrit par I.C. POPOFF et Coll. (J. Org. Chem. 28, 2898 (1963)) peuvent être mis à réagir avec les dérivés carbonylés III en l'absence de solvant et de catalyseur, l'eau formée au cours de la réaction, étant éliminés à la fin de l'opération par des moyens appropriés. La condensation peut s'effectuer avantageusement dans un solvant tel qu'un hydrocarbure aromatique par exemple le toluène ou un alcool par exemple l'éthanol dans lesquels il est possible d'éliminer l'eau par distillation azéotropique. Le condensation peut encore être avantageusement réalisée (sur le plan de la vitesse) en présence de quantités catalytiques d'un acide minéral ou organique comme par exemple l'acide paratoluènesulfonique. La température à laquelle on effectue cette tranformation est variable mais se situe très généralement entre 20 et 120°C.

b) c) la base $B^{\ominus}$ $M^{\oplus}$ mise en œuvre dans ces stades peut être un hydrure de métal alcalin, notamment les hydrures de sodium, de lithium ou de potassium, un composé alkyl-métal alcalin, notamment les organolithiens tels que le n-butyllithium ou les organosodés. On peut aussi faire appel aux alcoolates de métal alcalin ou alcalinoterreux, tels que le méthylate de sodium, de lithium, de potassium, de magnésium, le tertiobutylate de potassium, le tertioamylate de sodium.

En général, on utilise un équivalent stoéchiométrique de la base $B^{\ominus}$ $M^{\oplus}$, voire un léger excès, par exemple de 10% par rapport à l'équivalence. Mais il est également possible de mettre en œuvre des quantités de base inférieures voire nettement inférieures à l'équivalence stoéchiométrique.

Il convient de souligner, que, selon une variante de l'invention, quand on utilise un équivalent stoéchiométrique de la base $B^{\ominus}$ $M^{\oplus}$ il peut être avantageux d'éviter l'isolement du dérivé VII et de mettre à réagir directement VI avec le composé carbonylé VIII en évitant de la sorte également l'utilisation de la base $B'^{\ominus}$ $M'^{\oplus}$.

On opère généralement entre −78°C et +150°C, à une température choisie plus spécifiquement en fonction de la base $B^{\ominus}$ $M^{\oplus}$ dans une plage plutôt haut de gamme en particulier lors de la réalisation de la phase c.

Les solvants préférés sont les éthers linéaires ou cycliques tels que le tétrahydrofurane, les hydrocarbures, notamment les aromatiques tels que benzène, toluène, xylènes, les alcools, les amides notamment le diméthylformamide, les sulfoxides notamment le diméthylsulfoxide. Il peut être également avantageux, particulièrement quand on utilise des hydroxydes métalliques, d'opérer en système biphasique (eau – solvant tel que solvant halogéné comme par exemple le dichlorométhane ou hydrocarbure aromatique tels que benzène, toluène, xylènes) en présence d'un catalyseur de transfert de phase notamment un sel d'ammonium quaternaire tel que l'iodure de tétran-butylammonium ou un sel de phosphonium. Les traitements habituels permettant d'isoler le composé VII.

d) la base $B'^{\ominus}$ $M'^{\oplus}$ mise en œuvre dans la première partie de ce troisième stade, peut être choisie parmi celles énumérées au b) ci-dessus. En général, elle est utilisée en équivalence stoéchiométrique mais peut être en léger excès par exemple de 5 ou 10% par rapport à cette équivalence.

On opère généralement entre −20°C et +100°C avec une préférence pour le bas de gamme. Les solvants utilisés sont ceux décrits au stade b) ci-dessus.

Dans la seconde partie de ce stade, le composé carbonylé de formule VIII est mis à réagir avec le milieu réactionnel tel que défini ci-dessus à une température généralement similaire à celle de la première partie.

e) la réduction du dérivé de formule IX est effectuée avantageusement par un hydrure mixte de métal alcalin comme notamment un borohydrure tel que le borohydrure de sodium ou le borohydrure de potassium. La réduction est réalisée au sein d'un solvant inerte comme un éther tel que par exemple le tétrahydrorfurane ou le dioxanne ou encore dans un alcool tel que par exemple le méthanol ou l'éthanol.

Il peut être avantageux, dans certains cas et plus particulièrement dans les cas où le radical $R_2$ n'est pas un atome d'hydrogène, d'additionner au milieu réactionnel un équivalent molaire, par rapport au borohydrure mis en œuvre, d'un acide organi-

que comme par exemple l'acide acétique ou l'acide trifluoroacétique.

On peut également effectuer cette réduction par le biais d'un hydrogénation catalytique en phase homogène ou hétérogène dans des conditions bien connues d'une manière générale.

Les composés de formule I ainsi obtenus peuvent ensuite être isolés et purifiés selon les méthodes habituelles. Pour réaliser ces opérations, il peut être avantageux de transformer les composés libres de formule I en leurs sels, par exemple en leurs sels d'addition d'acides par réaction avec des acides minéraux ou organiques. A partir des sels, on peut libérer les composés de formule I selon les méthodes connues.

L'invention comprend également les produits intermédiaires obtenus aux différents stades de la synthèse:

- les composés de formule IV

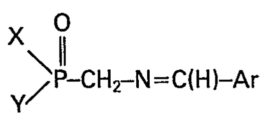

(IV)

- les composés de formule VII

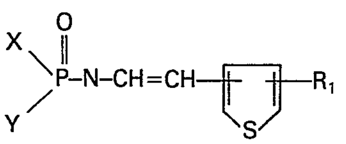

(VII)

- les composés de formule IX

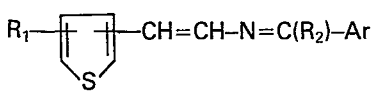

(IX)

- les composés de formule I

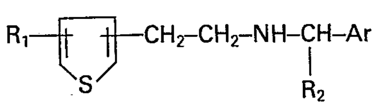

(I)

Les exemples suivants illustrent l'invention sans la limiter.

Exemple 1:

Préparation du chlorhydrate de la N-Orthochlorobenzyl, (thiényl-2)-2 éthylamine

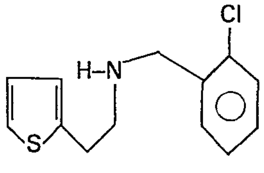

, HCl

- stade a.

N-thénylidène-2, aminométhylphosphonate de diéthyle

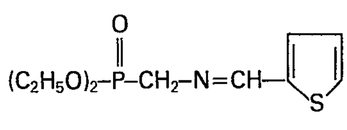

Une solution de 16,7 g (0,1 mole) d'aminométhylphosphonate de diéthyle dans 200 ml d'éthanol absolu est additionnée de 11,2 g (0,1 mole) de thénaldéhyde-2, portée au reflux puis évaporée en fournissant 26 g de N-(thénylidène-2), aminométhylphosphonate de diéthyle (rendement env. 100%) sous forme d'une huile jaune monotache en CCM (plaque de silice; éluant: acétate d'éthyle).

IR (film)
- C = N 1640 $cm^{-1}$
- P = O 1260 $cm^{-1}$
- P–O–C 1060 à 1080 $cm^{-1}$

RMN ($CDCl_3$) δ / TMS
- 1,35 ppm (t, 6 H)
- 3,9 à 4,45 ppm (m, 6 H)
- 7 à 7,8 ppm (m, 3 H)
- 8,5 ppm (d, 1 H)

- stades b, c, d.

(orthochlorophényl)-1, (thiényl-2)-4, aza-2, butadiène 1,3

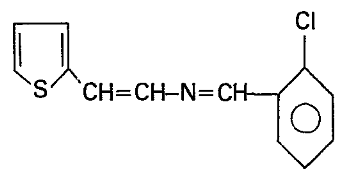

A une suspension de 11,2 g (0,1 mole) de terbutylate de potassium dans 160 ml de THF*, on ajoute goutte à goutte une solution de 27,9 g (0,1 mole) de N-(thénylidène-2), aminométhylphosphonate d'éthyle dans 40 ml de THF*. Au cours de l'addition, la température s'élève de 20 à 35°C. A la fin de l'addition, on porte le milieu à 40 à 45°C pendant 30 minutes puis on ajoute goutte à goutte un solution de 14,05 g (0,1 mole) d'orthochlorobenzaldéhyde dans 10 ml de THF. On laisse la réaction se poursuivre pendant une heure puis on évapore le THF. Le résidu est repris par de l'éther et de l'eau, et la phase aqueuse est reextraite par de l'éther. Les phases éthérées réunies, lavées à l'eau, séchées sur sulfate de sodium et évaporées fournissent 17,8 g (rendement 72%) d'(orthochlorophényl)-1, (thiényl-2)-4, aza-2, butadiène 1,3 sous forme d'une huile orangée, utilisée telle que dans le stade suivant.

* THF = Tetrahydrofurane

IR (film) C = N 1640 $cm^{-1}$

RMN ($CDCl_3$)
<u>CH</u> = N
- 8,6 ppm (s, 1 H)
- 8 ppm (m, 1 H)
- 6,9 à 7,9 ppm (m, 8 H)

- stade e.

chlorhydrate de la N-orthochlorobenzyl, (thiényl-2)-2 éthylamine

A une solution de 10,2 g (0,15 mole) de borohydrure de sodium dans 160 ml d'éthanol, on ajoute goutte à goutte l'azadiène brut précédent (17,8 g) en solution dans 40 ml d'éthanol.

Après la fin de l'addition, au cours de laquelle la température s'élève de 20 à 30°C, on abandonne le milieu à température ambiante pendant deux heures puis on le porte durant une heure à 45 à

50°C. Le milieu réactionnel est ensuite évaporé et le résidu repris par de l'eau et de l'éther isopropylique. La phase aqueuse est reextraite à l'éther puis les phases éthérées réunies sont lavées à l'eau, séchées sur sulfate de sodium et évaporées. A la base brute obtenue et mise en suspension dans 50 ml d'eau, on ajoute goutte à goutte à 50°C, 8,5 ml d'acide chlorhydrique aqueux 12N puis on porte le mélange à 90°C. La solution homogène obtenue traitée au noir animal puis filtrée, laisse précipiter par refroidissement des cristaux que l'on filtre, lave à l'eau permutée puis sèche à 50°C sous vide. On obtient ainsi 15,9 g (rendement 55% par rapport à l'aminométhylphosphonate de diéthyle engagé) au stade a) de chlorhydrate de N-orthochlorobenzyl, (thiényl-2)-2 éthylamine sous forme de cristaux blancs.

F = 143°C.

IR (pastille KBr): 3400 $cm^{-1}$, 2900 à 2600 $cm^{-1}$, 1575 $cm^{-1}$, 1450 $cm^{-1}$

RMN (DMSO $d_6$) δ / TMS
- 7 à 7,8 ppm (m, 8 H)
- 3,35 ppm (s, 4 H)
- 4,15 ppm (s, 2 H)
- env. 9 ppm (m, 2 H) échangeable avec $D_2O$

| Analyse: | $C_{13}H_{14}Cl$ NS, HCl = 288,35 | | |
|---|---|---|---|
| | C % | H % | N % |
| Calculé: | 54,16 | 5,24 | 4,85 |
| Trouvé: | 54,11 | 5,28 | 4,80 |

Exemple 2:

Oxalate de la N-Orthochlorobenzyl, [terbutoxy-5 (thiényl-2)]-2 éthylamine

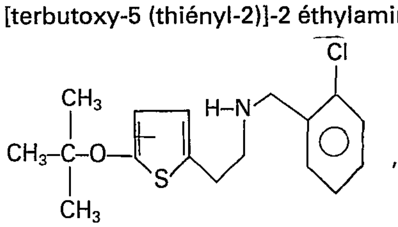
, HOOC–COOH

• stade a.

N [terbutoxy-5 thénylidène-2], aminométhylphosphonate de diéthyle

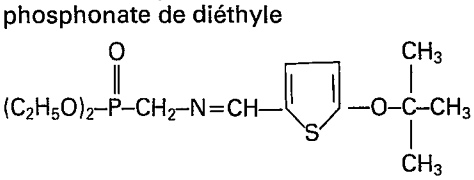

En opérant comme décrit à l'exemple 1, à partir de 18,4 g (0,1 mole) de terbutoxy-5 thénaldéhyde-2 et 16,7 g (0,1 mole) d'aminométhylphosphonate de diéthyle, on obtient 33,3 g (rendement 100%) d'imine référencée.

IR (film): 3000 $cm^{-1}$, 1630 $cm^{-1}$, 1250 $cm^{-1}$, 1050 $cm^{-1}$

RMN ($CDCl_3$) δ / TMS
- 1,3 ppm (m, 15 H)
- 4 ppm (m, 6 H)
- 6,2 ppm (d, 1 H) J = 4 Hz } système AB
- 6,7 ppm (d, 1 H) J = 4 Hz }
- 8,3 ppm (d, 1 H) J = 2 Hz

• stades b, c, d.

(chloro-2 phényl)-1 [terbutoxy-5 (thiényl-2)]-4, aza-2, butadiène 1,3

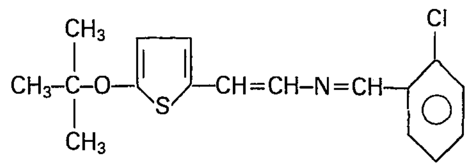

A une solution de 16,65 g (0,05 mole) de N-[terbutoxy-5 (thénylidène-2)] aminométhylphosphonate de diéthyle, dans 100 ml de THF, on ajoute goutte à goutte et en maintenant la température entre 25 et 30°C, 17,85 g (0,05 mole) d'une solution 2,8M de n-butyllithium dans l'hexane. 30 minutes après la fin de l'addition, on additionne goutte à goutte 7 g (0,05 mole) d'orthochlorobenzaldéhyde dans 10 ml de THF puis on porte le milieu réactionnel durant 1 h à 45 à 50°C. A la fin de cette période, le THF est évaporé et le résidu traité comme dans l'exemple 1, fournit 10,6g (66,5%) d'une huile jaune orangée que l'on utilise telle que dans le stade suivant.

• stade e.

Oxalate de la N-orthochlorobenzyl [terbutoxy-5 (thiényl-2)]-2 éthylamine

A partir de 10,6 g (33 m mole) de l'aza-2 butadiène-1,3 préparé ci-dessus et de 5,1 g (75 m mole) de borohydrure de sodium et en opérant comme à l'exemple 1 dans 100 ml d'éthanol, on obtient 10,7 g de l'amine référencée sous forme d'une huile jaune. Cette huile en solution dans 50 ml d'acétone est additionnée goutte à goutte à une solution de 3,15 g (35 m mole) d'acide oxalique dans 50 ml d'acétone. Après deux heures d'agitation à température ambiante, le précipité formé est filtré, rincé à l'acétone puis à l'éther isopropylique et finalement séché sous vide à température ambiante. On obtient ainsi 10,4g (rendement 50% par rapport à l'aminométhylphosphonate de diéthyle) d'oxalate de N-orthochlorobenzyl [terbutoxy-5 (thiényl-2)]-2 éthylamine sous forme de cristaux blancs.

F = 202°C (dec.).

IR (film, sur la base): 3300 $cm^{-1}$, 2850 à 3000 $cm^{-1}$, 1560 $cm^{-1}$, 1150 $cm^{-1}$

RMN ($CDCl_3$) δ / TMS
- 1,3 ppm (s, 9 H) $(C\underline{H}_3)_3C$
- 1,7 ppm (s, 1 H) échangeable avec $D_2O$
- 2,8 ppm (s, 4 H)

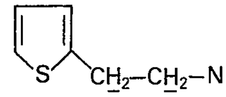

- 3,85 ppm (s, 2 H) $-N-C\underline{H}_2-Ar$
- 6,05 ppm (d, 1 H)
- 6,35 ppm (d, 1 H)
- 7,2 ppm (m, 4 H)

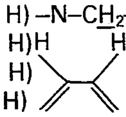
système AB avec JAB = 4 Hz

| | | | |
|---|---|---|---|
| Analyse: | $C_{17}H_{22}Cl\ NOS,C_2H_2O_4$ M = 413,917 | | |
| | C % | H % | N % |
| Calculé: | 55,13 | 5,81 | 3,38 |
| Trouvé: | 55,25 | 5,75 | 3,36 |

Exemple 3:

Oxalate de la N-furfuryl-2, (thiényl-2)-2 éthylamine

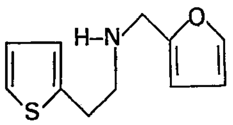

, HOOC–COOH

• stade a.

N-thénylidène-2, aminométhylphosphonate de diéthyle

On prépare 0,1 mole de produit référencé en opérant comme décrit à l'exemple 1.

• stades b, c, d.

(furyl-2)-1, (thiényl-2)-4, aza-2, butadiène-1,3

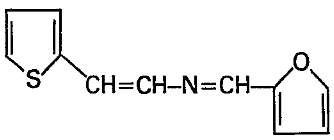

A une suspension de 4,8 g (0,1 mole) d'hydrure de sodium (à 50% dans de l'huile) dans 100 ml de THF, on additionne goutte à goutte une solution de 26,1 g (0,1 mole) de N-(thénylidène-2) aminométhylphosphonate de diéthyle dans 40 ml de THF. Après la fin de l'addition, durant laquelle la température est passée de 20 à 30°C, on porte le milieu à 45°C pendant deux heures puis on y ajoute goutte à goutte une solution de 9,6 g (0,1 mole) de furfural dans 20 ml de THF. On maintient ensuite le milieu, sous agitation, à 45 à 50°C durant deux heures puis on traite le milieu réactionnel comme décrit à l'exemple 1.

On obtient ainsi 17,85 g (rendement 88%) d'aza-2 butadiène-1,3, référencé sous forme d'une huile orangée que l'on met en œuvre telle que dans le stade suivant.

• stade e.

Oxalate de la N-furfuryl-2, (thiényl-2)-2 éthylamine

L'azadiène obtenu ci-dessus en solution dans 200 ml d'éthanol est traité avec 6,7 g (0,176 mole) de borohydrure de sodium comme décrit à l'exemple 2. La base brute obtenue mise en solution dans 50 ml d'acétone est additionnée à une solution de 8 g d'acide oxalique dans 50 ml d'acétone. Après deux heures d'agitation à température ambiante, le précipité est filtré, rincé à l'acétone puis recristallisé dans un mélange eau-éthanol (60–40); on obtient finalement 17,52 g (rendement 59% par rapport à l'aminométhylphosphonate de diéthyle) d'oxalate de N-furfuryl-2, (thiényl-2)-2 éthylamine sous forme de cristaux.

F = 215°C.

IR (pastille KBr)
$3400\ cm^{-1}$, $3040\ cm^{-1}$,
$2850\ cm^{-1}$, $1715\ cm^{-1}$,
$1650\ cm^{-1}$, $1480\ cm^{-1}$

RMN ($CDCl_3$, sur base libérée de l'oxalate) δ / TMS

1,65 ppm (s, 1 H) échangeable avec $D_2O$

2,8 ppm (s, 4 H) 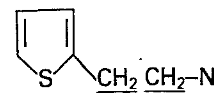

3,65 ppm (s, 2 H) 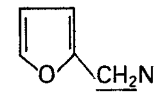

6,1 ppm (m, 2 H) 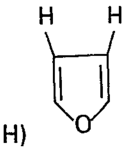

6,6 à 7,3 ppm (m, 4 H)

| | | | |
|---|---|---|---|
| Analyse: | $C_{11}H_{13}NOS,\ C_2H_2O_4$ = 297,324 | | |
| | C % | H % | N % |
| Calculé: | 52,52 | 5,05 | 4,71 |
| Trouvé: | 52,45 | 5,01 | 4,63 |

Exemple 4:

Chlorhydrate de la N-Orthochlorobenzyl, (thiényl-2)-2 éthylamine

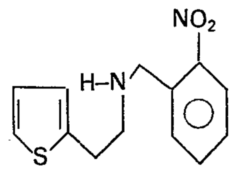

, HCl

• stade a.

N-thénylidène-2, aminométhylphosphonate de diéthyle

On prépare 0,1 mole de produit référencé en opérant comme décrit à l'exemple 1.

• stades b, c, d.

(orthonitrophényl)-1, (thiényl-2)-4, aza-2, butadiène-1,3

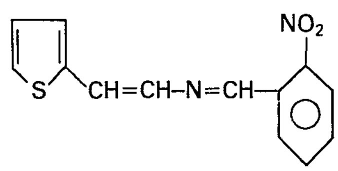

En opérant comme décrit à l'exemple 1, on obtient 22 g (rendement 85%) d'azadiène référencé sous forme d'une huile orangée que l'on utilise telle que dans le stade suivant.

• stade e.

chlorhydrate de N-orthonitrobenzyl (thiényl-2)-2 éthylamine

A l'azadiène obtenu ci-dessus, en solution dans 200 ml d'éthanol, sont additionnés par petites portions 11,56 g (0,17 mole) de borohydrure de sodium, en maintenant la température inférieure à

25°C. Le milieu réactionnel est ensuite agité deux heures à température ambiante puis versé dans un litre d'eau et extrait au chloroforme.

La phase organique lavée à l'eau, séchée sur sulfate de sodium puis évaporée fournit la base sous forme d'une huile qui est chlorhydratée dans l'éthanol. Le précipité formé est recristallisé dans l'éthanol. On obtient ainsi 18,2 g (rendement 61% par rapport à l'aminomethylphosphonate de diéthyle) de chlorhydrate de N-orthonitrobenzyl, (thiényl-2)-2 éthylamine sous forme de cristaux blancs.

F = 168°C.

IR (pastille KBr): 3450 $cm^{-1}$, 3000 à 2900 $cm^{-1}$, 2700 $cm^{-1}$, 1560 à 1525 $cm^{-1}$, 1450 $cm^{-1}$, 1340 $cm^{-1}$

RMN ($CDCl_3$, sur base libérée du chlorhydrate)
δ / TMS
1,65 ppm (s, 1 H) échangeable avec $D_2O$
2,9 ppm (t, 4 H) 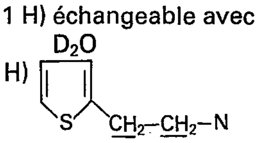
4 ppm (s, 2 H) N–$CH_2$–Ar
6,7 à 7,9 ppm (m, 7 H) Aromatiques

| Analyse: | $C_{13}H_{14}N_2O$ S, HCl = 298,773 | | |
|---|---|---|---|
| | C % | H % | N % |
| Calculé: | 52,26 | 5,06 | 9,38 |
| Trouvé: | 52,28 | 5,03 | 9,31 |

Exemple 5:
N-(picolyl)-4, (thiényl-2)-2 éthylamine

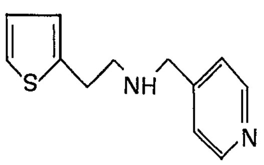

• stade a.
N-(thénylidène-2), aminométhylphosphonate de diéthyle

On prépare 0,1 mole de produit référencé en opérant comme décrit à l'exemple 1.

• stades b, c, d.
(pyridyl-4)-1, (thiényl-2)-4, aza-2, butadiène-1,3

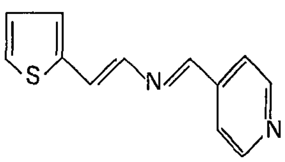

En opérant comme à l'exemple 1, on obtient à partir de 0,1 mole de N-(thénylidène-2), aminométhylphosphonate de diéthyle et de 0,1 mole de pyridyl-4 carboxaldéhyde, 18 g (rendement 85%) d'azadiène référencié sous forme d'une huile orangée que l'on utilise telle quelle dans l'étape suivante.

Un échantillon pur de l'azadiène est obtenu par chromatographie sur silice (éluant: acétate d'éthyle/hexane 50/50).

Les cristaux orangés obtenus ont les caractéristiques suivantes:

F = 165°C.

RMN ($CDCl_3$) δ / TMS
6,5 à 7,5 ppm (m, 7 H)
8,4 ppm (s, 1 H) CH = N
8,45 ppm (d, 2 H) partie A du système AB de la pyridine

IR (pastille KBr): 1600 $cm^{-1}$, 1560 $cm^{-1}$, 1420 $cm^{-1}$

• stade e.
N-(picolyl-4), (thiényl-2)-2 éthylamine

L'azadiène brut obtenu ci-dessus est réduit au borohydrure de sodium dans les conditions décrites à l'exemple 5 pour fournir, après purification par chromatographie sur silice 9,16 g (rendement 42% par rapport à l'aminométhylphosphonate de diéthyle) du produit référencé sous forme d'une huile jaune claire qui brunit à l'air.

IR (film): 3300 $cm^{-1}$, 2900 $cm^{-1}$, 1600 $cm^{-1}$, 1440 $cm^{-1}$,

RMN ($CDCl_3$) δ / TMS
1,7 ppm (s, 1 H) échangeable avec $D_2O$
3 ppm (t, 4 H) Ar–$CH_2$–$CH_2$–N
3,8 ppm (s, 2 H) Ar–$CH_2$–N
6,6 à 7,5 ppm (m, 5 H)
8,4 ppm (d, 2 H)

Exemple 6:
Chlorhydrate de la N-thényl-2 (thiényl-2)-2 éthylamine

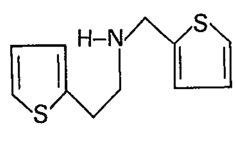 , HCl

• stade a.
N-thénylidène-2, aminométhylphosphonate de diisopropyle

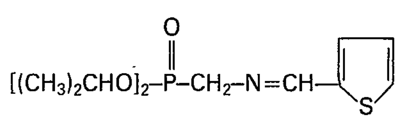

On prépare 0,1 mole de produit référencé en opérant comme décrit à l'exemple 1.

IR (film): 1635 $cm^{-1}$, 1260 $cm^{-1}$, 1080 à 1060 $cm^{-1}$

RMN ($CDCl_3$) δ / TMS
- 1,3 ppm (d, 12 H)
- 4,05 ppm (d, 2 H) P–$\underline{CH_2}$ N=
- 4,75 ppm (m, 2 H)
- 7 à 7,6 ppm (m, 3 H)
- 8,35 ppm (d, 1 H) Ar–$\underline{CH}$=N

• stades b, c, d.
(thiényl-2)-1, (thiényl-2)-4, aza-2, butadiène-1,3

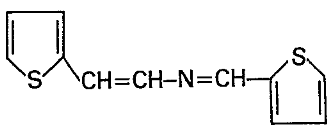

En opérant comme décrit à l'exemple 1, on obtient après recristallisation dans le méthanol, 13,6 g (rendement 62%) d'azadiène référencé sous forme de cristaux jaunes.

F = 163°C.

IR (pastille KBr) { C = N 1635 $cm^{-1}$

RMN ($DMSOd_6$) δ / TMS
- 8,35 ppm (s, 1 H)
- 6,9 à 7,5 ppm (m, 8 H)

Analyse: $C_{11}H_9NS_2$ = 219,32

| | C % | H % | N % |
|---|---|---|---|
| Calculé: | 60,27 | 4,10 | 6,39 |
| Trouvé: | 60,25 | 4,07 | 6,40 |

• stade e.
Chlorhydrate de la N-thényl-2, (thiényl-2)-2 éthylamine

En opérant comme décrit à l'exemple 1, sur 10,95 g (0,05 mole) d'azadiène préparé ci-dessus, on obtient, après chlorhydratation dans l'éther isopropylique, 11,15 g (rendement 53% par rapport à l'aminométhylphosphonate de diisopropyle) de chlorhydrate de N-thényl-2, (thiényl-2)-2 éthylamine sous forme de cristaux blancs.

F = 230°C (dec.).

IR (pastille KBr)
- 3400 $cm^{-1}$
- 2920 $cm^{-1}$
- 2750 $cm^{-1}$
- 1440 $cm^{-1}$
- 1250 $cm^{-1}$

RMN ($DMSOd_6$) δ / TMS
- 6,9 à 7,5 ppm (m, 6 H)
- 4,40 ppm (s, 2 H)
- 3,2 ppm (m, 4 H)
- env. 9 ppm (m, 2 H) échangeable avec $D_2O$

Analyse: $C_{11}H_{13}NS_2$, HCl = 259,815

| | C % | H % | N % |
|---|---|---|---|
| Calculé: | 50,86 | 5,39 | 5,39 |
| Trouvé: | 50,90 | 5,40 | 5,37 |

Exemple 7:
Préparation du chlorhydrate de la N-orthochlorobenzyl, (thiényl-3)-2 éthylamine

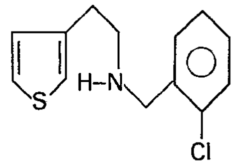 , HCl

• stade a.
N-thénylidène-3, aminométhylphosphonate de diéthyle

On prépare 0,1 mole de produit référencé en opérant comme décrit à l'exemple 1.

IR (film)
- 1635 $cm^{-1}$
- 1250 $cm^{-1}$
- 1050 $cm^{-1}$

RMN ($CDCl_3$) δ / TMS
- 1,3 ppm (t, 6 H)
- 4 ppm (m, 6 H)
- 7,2 à 7,6 ppm (m, 3 H)
- 6,3 ppm (d, 1 H)

• stades b, c, d.
(orthochlorophényl)-1, (thiényl-3)-4, aza-2, butadiène-1,3

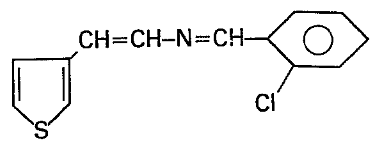

En poursuivant comme à l'exemple 1, mais en mettant en œuvre le orthochlorobenzaldehyde, on obtient après traitement 17,3 g (rendement 71%) d'aza-2 butadiène-1,3 sous forme d'une huile jaune que l'on met en réaction telle que dans le stade suivant.

• stade e.
chlorhydrate de N-orthochlorobenzyl (thiényl-3)-2 éthylamine

En opérant comme à l'exemple 1, sur le produit préparé ci-dessus, on obtient 15,1 g (rendement 52% par rapport à l'aminométhylphosphonate engagé au stade a) de chlorhydrate référencé sous forme de cristaux blancs.

F = 176°C.

IR (pastille KBr) { 3400 $cm^{-1}$, 2900 $cm^{-1}$, 2800 à 2700 $cm^{-1}$, 1575 $cm^{-1}$, 1450 $cm^{-1}$

RMN ($DMSOd_6$) δ / TMS
- 3,2 ppm (s, 4 H)
- 4,05 ppm (s, 2 H)
- 6,9 à 7,8 ppm (m, 7 H)
- env. 9 ppm (m, 2 H) échangeable avec $D_2O$

| Analyse: | $C_{13}H_{14}Cl\ NS, HCl$ = 288,236 | | |
|---|---|---|---|
| | C % | H % | N % |
| Calculé: | 54,16 | 5,24 | 4,85 |
| Trouvé: | 54,25 | 5,20 | 4,79 |

Exemple 8:
Préparation du chlorhydrate de la N'orthochlorobenzyl, (thiényl-2)-2 éthylamine

• stade a.
N-thiénylidène-2, aminométhyl, phénylphosphinate d'isopropyle

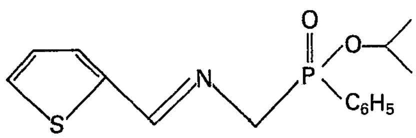

On prépare 0,1 mole de produit référencé en opérant comme décrit à l'exemple 1.

IR (film) C = N
1625 $cm^{-1}$
1430 $cm^{-1}$
1200 $cm^{-1}$
980 $cm^{-1}$

RMN ($CDCl_3$) δ / TMS
1,4 ppm (d de d, 6 H)
4,15 ppm (d, 2 H)
4,75 ppm (m, 1 H)
7 à 8 ppm (m, 8 H)
8,25 ppm (d, 1 H)

• stades b, c.
phénylphosphinate d'isopropyle, N-[béta-(thiényl-2) vinyl] amide

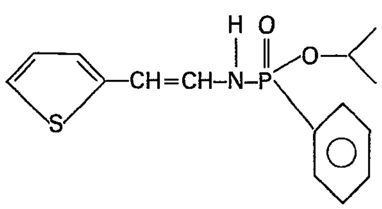

A partir de 0,1 mole d'imine préparée ci-dessus et en opérant comme à l'exemple 1, on obtient 18,6 g (rendement 60,5%) du produit fini référencé sous forme de cristaux.
F = 125°C.

IR (pastille KBr)
3400 à 3150 $cm^{-1}$
1650 $cm^{-1}$
1220 $cm^{-1}$
1000 $cm^{-1}$

RMN ($CDCl_3$) δ / TMS
1,35 ppm (d, 6 H)
4,8 ppm (m, 1 H)
5,9 ppm (m, 1 H)
6,2 à 7 ppm (m, 4 H)
7 à 8 ppm (m, 6 H) dont 1 échangeable avec $D_2O$

• stade d.
orthochlorophényl-1, (thiényl-2)-4 aza-2, butadiène-1,3

A une suspension de (0,06 mole) d'hydrure de sodium (à 50% dans l'huile) dans 50 ml de THF, on ajoute goutte à goutte une solution de 0,06 mole du phosphinate préparé au stade précédent dans 20 ml de THF. A la fin de l'addition, la température du milieu réactionnel est portée à 40 à 45°C pendant 30 minutes, puis on ajoute goutte à goutte une solution de 0,06 mole d'orthochlorobenzaldéhyde dans 20 ml de THF. On poursuit l'agitation pendant 1 heure à 40 à 45°C puis après refroidissement, on verse le milieu réactionnel dans l'eau et on extrait à l'éther isopropylique. Les phases organiques sont lavées avec une solution aqueuse saturée de chlorure de sodium, séchées sur sulfate de sodium et évaporées pour fournir 14,1 g (rendement 95% par rapport au phosphinate engagé) de l'azadiène de référence.

Un échantillon purifié sur colonne de silice (éluant:hexane 95% – acétate d'éthyle 5%) montre que le produit obtenu est identique à celui préparé dans l'exemple 1.
IR et RMN identiques.
Mêmes Rf en CPL, CPG, CCM.

• stade e.
Chlorhydrate de la N-orthochlorobenzyl, (thiényl-2)-2 éthylamine

La réduction de l'azadiène obtenu au stade précédent dans les conditions décrites à l'exemple 1, fournit, après traitements similaires 14,7 g (rendement 51% par rapport à l'aminométhylphosphinate) de chlorhydrate d'orthochlorobenzyl, (thiényl-2)-2 éthylamine dont les caractéristiques physiques, spectrales et analytiques sont identiques à celles du produit obtenu à l'exemple 1.

**Revendications pour les Etats contractants BE, CH, DE, FR, GB, IT, LI, LU, NL, SE**

1. Procédé de préparation de dérivés de (thiényl-2)- et (thiényl-3)-2 éthylamines de formule

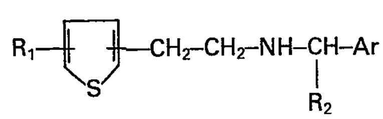 (I)

dans laquelle $R_1$ en position 2, 3, 4 ou 5 représente un atome d'hydrogène ou d'halogène, un radical nitro, amino, un radical alkyle ou alcoxy linéaire ou ramifié; $R_2$ représente un atome d'hydrogène ou un radical alkyle linéaire ou ramifié; Ar représente un radical thiényle, furyle, pyridyle, phényle ou naphtyle, ou phényle mono- ou polysubstitué par des groupes tels que halogène, nitro, cyano, amino, carboxy, alkyle, alcoxy ou phényle, caractérisé en ce qu'on condense un dérivé de formule

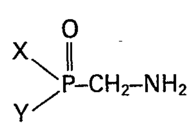 (II)

dans lequel X et Y sont identiques ou différents et représentent un radical alkyle, alcoxy, aryle, aryloxy, diaryl – ou dialkylamino avec un composé carbonylé de formule

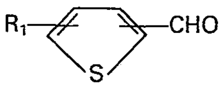

(III)

dans lequel $R_1$ est tel que défini précédemment pour obtenir un composé de formule

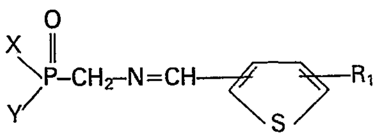

(IV)

dans lequel X, Y et $R_1$ sont tels que définis précédemment, qui est traité par une base $B^{\ominus}$ $M^{\oplus}$ pour conduire à un carbanion de formule

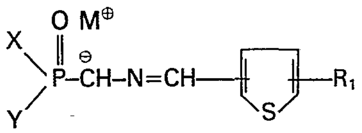

(V)

dans lequel X, Y et $R_1$ sont tels que définis précédemment, qui se transforme, sous l'action de la chaleur, en dérivé de formule

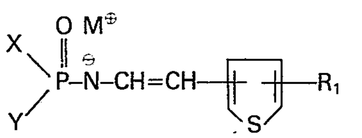

(VI)

puis, après reprise par l'eau, en dérivé de formule

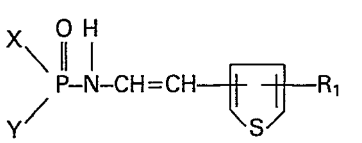

(VII)

que l'on traite successivement par une base $B'^{\ominus}$ $M'^{\oplus}$, puis par un composé carbonylé de formule

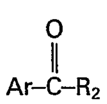

(VIII)

sans lequel Ar et $R_2$ sont tels que définis précédemment pour obtenir le dérivé de formule

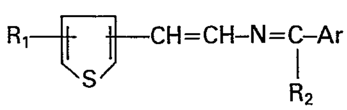

(IX)

puis par traitement par un agent réducteur en composé de formule I.

2. Procédé suivant la revendication 1, caractérisé en ce que la réaction entre le composé organophosphoré de formule II et le dérivé carbonylé de formule III est catalysée par la présence d'un acide minéral ou organique tel que l'acide paratoluènesulfonique.

3. Procédé suivant la revendication 1, caractérisé en ce que la transformation du carbanion de formule (V) en dérivé de formule (VII) s'effectue en une seule opération (one pot) sans isolement du dérivé intermédiaire de formule (VI).

4. Procédé suivant la revendication 3, caractérisé en ce que la réaction s'effectue à une température comprise entre −78°C et +150°C.

5. Procédé selon la revendication 1, caractérisé en ce que la base $B^{\ominus}$ $M^{\oplus}$ est un hydrure de métal alcalin, un composé alkyl-métal alcalin ou un alcoolate de métal alcalin ou alcalino-terreux.

6. Procédé selon la revendication 5, caractérisé en ce que $M^{\oplus}$ est un métal choisi parmi le sodium, le lithium, le potassium et le magnésium.

7. Procédé selon la revendication 1, caractérisé en ce que la base $B'^{\ominus}$ $M'^{\oplus}$ est analogue à la base $B^{\ominus}$ $M^{\oplus}$ définie à la revendication 5 ou 6.

8. Procédé suivant la revendication 1, caractérisé en ce que la réduction du dérivé de formule (IX) est effectuée par un hydrure mixte de métal alcalin, notamment le borohydrure de sodium ou le borohydrure de potassium.

9. La N-orthochlorobenzyl [terbutoxy-5- (thiényl-2)]-2 éthylamine.

10. La N-furfuryl-2 (thiényl-2) éthylamine.

11. La N-orthochlorobenzyl (thiényl-2)-2 éthylamine.

12. La N-(picolyl-4) (thiényl-2)-2 éthylamine.

13. La N-(thényl-2)- (thiényl-2)-2 éthylamine.

14. La N-orthochlorobenzyl (thiényl-3)-2 éthylamine.

**Revendications pour l'Etat contractant AT**

1. Procédé de préparation de dérivés de (thiényl-2)- et (thiényl-3)-2 éthylamines de formule

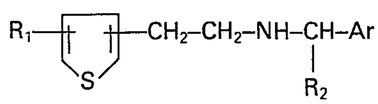

(I)

dans laquelle $R_1$ en position 2, 3, 4 ou 5 représente un atome d'hydrogène ou d'halogène, un radical nitro, amino, cyano, carboxyle, un radical alkyle ou alcoxy linéaire ou ramifié; $R_2$ représente un atome d'hydrogène ou un radical alkyle linéaire ou ramifié; Ar représente un radical thiényle, furyle, pyridyle, phényle ou naphtyle, ou phényle mono- ou polysubstitué par des groupes tels que halogène, nitro, cyano, amino, carboxy, alkyle, alcoxy ou phényle, caractérisé en ce qu'on condense un dérivé de formule

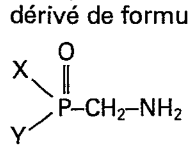

(II)

dans lequel X et Y sont identiques ou différents et représentent un radical alkyle, alcoxy, aryle, aryloxy, diaryl – ou dialkylamino avec un composé carbonylé de formule

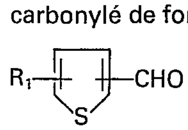

(III)

dans lequel $R_1$ est tel que défini précédemment pour obtenir un composé de formule

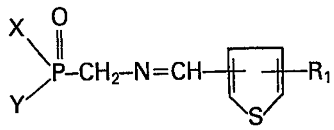

(IV)

dans lequel X, Y et $R_1$ sont tels que définis précédemment, qui est traité par une base $B^{\ominus}$ $M^{\oplus}$ pour conduire à un carbanion de formule

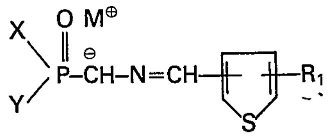

(V)

dans lequel X, Y et $R_1$ sont tels que définis précédemment, qui se transforme, sous l'action de la chaleur, en dérivé de formule

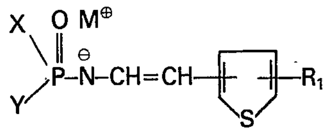

(VI)

puis, après reprise par l'eau, en dérivé de formule

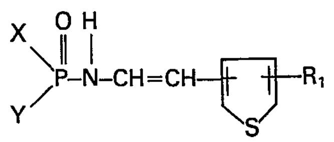

(VII)

que l'on traite successivement par une base $B'^{\ominus}$ $M'^{\oplus}$, puis par un composé carbonylé de formule

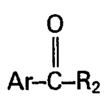

(VIII)

sans lequel Ar et $R_2$ sont tels que définis précédemment pour obtenir le dérivé de formule

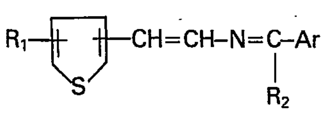

(IX)

puis par traitement par un agent réducteur en composé de formule I.

2. Procédé suivant la revendication 1, caractérisé en ce que la réaction entre le composé organophosphoré de formule II et le dérivé carbonylé de formule III est catalysée par la présence d'un acide minéral ou organique tel que l'acide paratoluènesulfonique.

3. Procédé suivant la revendication 1, caractérisé en ce que la transformation du carbanion de formule (V) en dérivé de formule (VII) s'effectue en une seule opération (one pot) sans isolement du dérivé intermédiaire de formule (VI).

4. Procédé suivant la revendication 3, caractérisé en ce que la réaction s'effectue à une température comprise entre −78°C et +150°C.

5. Procédé selon la revendication 1, caractérisé en ce que la base $B^{\ominus}$ $M^{\oplus}$ est un hydrure de métal alcalin, un composé alkyl-métal alcalin ou un alcoolate de métal alcalin ou alcalino-terreux.

6. Procédé selon la revendication 5, caractérisé en ce que $M^{\oplus}$ est un métal choisi parmi le sodium, le lithium, le potassium et le magnésium.

7. Procédé selon la revendication 1, caractérisé en ce que la base $B'^{\ominus}$ $M'^{\oplus}$ est analogue à la base $B^{\ominus}$ $M^{\oplus}$ définie à la revendication 5 ou 6.

8. Procédé suivant la revendication 1, caractérisé en ce que la réduction du dérivé de formule (IX) est effectuée par un hydrure mixte de métal alcalin, notamment le borohydrure de sodium ou le borohydrure de potassium.

**Patentansprüche für die Vertragsstaaten: BE, CH, DE, FR, GB, IT, LI, LU, NL, SE**

1. Verfahren zur Herstellung von (2-Thienyl)- und 2-(3-Thienyl)-ethylaminen der Formel

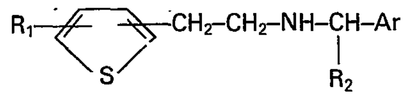

(I)

worin $R_1$ in 2-, 3-, 4- oder 5-Stellung ein Wasserstoffatom oder Halogenatom, einen Nitro-, Amino-, Cyano-, Carboxyl-, einen linearen oder verzweigten Alkyl- oder Alkoxyrest darstellt, $R_2$ ein Wasserstoffatom oder ein linearer oder verzweigter Alkylrest ist, Ar ein Thienyl-, Furyl-, Pyridyl-, Phenyl- oder Naphthylrest oder ein mit Gruppen wie Halogen, Nitro, Cyano, Amino, Carboxy, Alkyl, Alkoxy oder Phenyl einfach oder mehrfach substituierter Phenylrest ist, dadurch gekennzeichnet, dass man ein Derivat der Formel

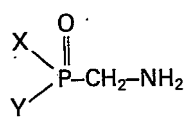

(II)

in der X und Y gleich oder verschieden sind und einen Alkyl-, Alkoxy-, Aryl-, Aryloxy-, Diaryl- oder Dialkylaminorest darstellen, mit einer Carbonylverbindung der Formel

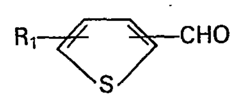

(III)

kondensiert, in der $R_1$ die vorstehende Bedeutung hat, um eine Verbindung der Formel

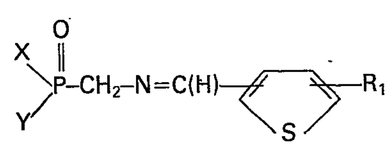

(IV)

zu erhalten, in der X, Y und $R_1$ die obige Bedeutung haben, die man mit einer Base $B^{\ominus}$ $M^{\oplus}$ behandelt, um zu einem Carbanion der Formel

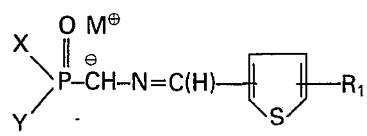

(V)

zu gelangen, in der X, Y und $R_1$ die obige Bedeutung haben, die man unter Einwirkung von Wärme in ein Derivat der Formel

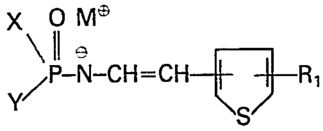
(VI)

umwandelt und dann nach Aufnahme in Wasser in ein Derivat der Formel

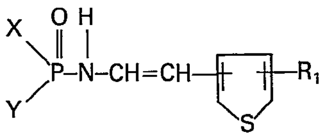
(VII)

das man nacheinander mit einer Base $B'^{\ominus} M'^{\oplus}$ und dann mit einer Carbonylverbindung der Formel

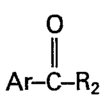
(VIII)

behandelt, in der Ar und $R_2$ die obige Bedeutung haben, um ein Derivat der Formel

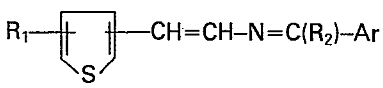
(IX)

zu erhalten und dann durch Behandlung mit einem Reduktionsmittel zur Verbindung der Formel I gelangt.

2. Verfahren nach Anspruch 1, dadurch gekennzeichnet, dass die Umsetzung zwischen der Organophosphorverbindung der Formel II und dem Carbonylderivat der Formel III durch Anwesenheit einer anorganischen oder organischen Säure wie p-Toluolsulfonsäure katalysiert wird.

3. Verfahren nach Anspruch 1, dadurch gekennzeichnet, dass die Umwandlung des Carbanions der Formel V zum Derivat der Formel VII in einem einzigen Arbeitsgang (ein Gefäss) ohne Isolierung des Zwischenderivats der Formel VI erfolgt.

4. Verfahren nach Anspruch 3, dadurch gekennzeichnet, dass die Reaktion bei einer zwischen −78°C und +150°C liegenden Temperatur durchgeführt wird.

5. Verfahren nach Anspruch 1, dadurch gekennzeichnet, dass die Base $B^{\ominus} M^{\oplus}$ ein Hydrid eines Alkalimetalls, eine Alkalimetallalkylverbindung oder ein Alkali- oder Erdalkalimetallalkoholat ist.

6. Verfahren nach Anspruch 5, dadurch gekennzeichnet, dass $M^{\oplus}$ ein aus Natrium, Lithium, Kalium und Magnesium ausgewähltes Metall ist.

7. Verfahren nach Anspruch 1, dadurch gekennzeichnet, dass die Base $B'^{\ominus} M'^{\oplus}$ analog der in Anspruch 6 definierten Base $B^{\ominus} M^{\oplus}$ ist.

8. Verfahren nach Anspruch 1, dadurch gekennzeichnet, dass die Reduktion des Derivats der Formel IX mit einem gemischten Alkalimetallhydrid, insbesondere Natriumborhydrid oder Kaliumborhydrid durchgeführt wird.

9. N-o-Chlorobenzyl-2[5-t-butoxy-(2-thienyl)]-ethylamin.

10. 2-N-Furfuryl-2-(2-thienyl)-ethylamin.

11. N-o-Nitrobenzyl-2-(2-thienyl)-ethylamin.

12. N-(4-Picolyl)-2-(2-thienyl)-ethylamin.

13. N-(2-thenyl)-2-(2-thienyl)-ethylamin.

14. N-o-Chlorobenzyl-2-(3-thienyl)-ethylamin.

**Patentansprüche für den Vertragsstaat: AT**

1. Verfahren zur Herstellung von (2-Thienyl)- und 2-(3-Thienyl)-ethylaminen der Formel

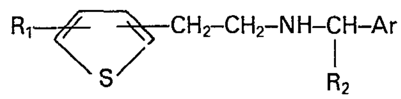
(I)

worin $R_1$ in 2-, 3-, 4- oder 5-Stellung ein Wasserstoffatom oder Halogenatom, einen Nitro-, Amino-, einen linearen oder verzweigten Alkyl- oder Alkoxyrest darstellt, $R_2$ ein Wasserstoffatom oder ein linearer oder verzweigter Alkylrest ist, Ar ein Thienyl-, Furyl-, Pyridyl-, Phenyl- oder Naphthylrest oder ein mit Gruppen wie Halogen, Nitro, Cyano, Amino, Carboxy, Alkyl, Alkoxy oder Phenyl einfach oder mehrfach substituierter Phenylrest ist, dadurch gekennzeichnet, dass man ein Derivat der Formel

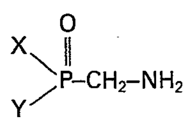
(II)

in der X und Y gleich oder verschieden sind und einen Alkyl-, Alkoxy-, Aryl-, Aryloxy-, Diaryl- oder Dialkylaminorest darstellen, mit einer Carbonylverbindung der Formel

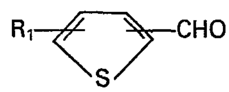
(III)

kondensiert, in der $R_1$ die vorstehende Bedeutung hat, um eine Verbindung der Formel

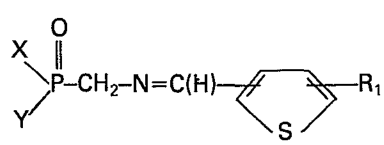
(IV)

zu erhalten, in der X, Y und $R_1$ die obige Bedeutung haben, die man mit einer Base $B^{\ominus} M^{\oplus}$ behandelt, um zu einem Carbanion der Formel

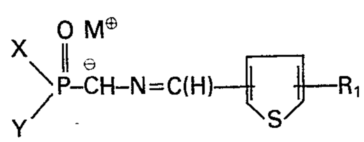
(V)

zu gelangen, in der X, Y und $R_1$ die obige Bedeutung haben, die man unter Einwirkung von Wärme in ein Derivat der Formel

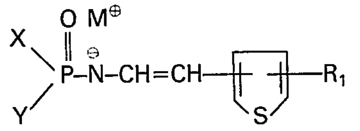

(VI)

umwandelt und dann nach Aufnahme in Wasser in ein Derivat der Formel

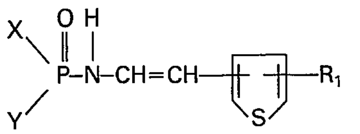

(VII)

das man nacheinander mit einer Base $B'^{\ominus}$ $M'^{\oplus}$ und dann mit einer Carbonylverbindung der Formel

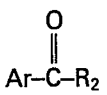

(VIII)

behandelt, in der Ar und $R_2$ die obige Bedeutung haben, um ein Derivat der Formel

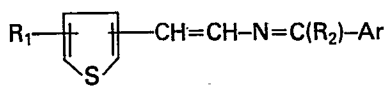

(IX)

zu erhalten und dann durch Behandlung mit einem Reduktionsmittel zur Verbindung der Formel I gelangt.

2. Verfahren nach Anspruch 1, dadurch gekennzeichnet, dass die Umsetzung zwischen der Organophosphorverbindung der Formel II und dem Carbonylderivat der Formel III durch Anwesenheit einer anorganischen oder organischen Säure wie p-Toluolsulfonsäure katalysiert wird.

3. Verfahren nach Anspruch 1, dadurch gekennzeichnet, dass die Umwandlung des Carbanions der Formel V zum Derivat der Formel VII in einem einzigen Arbeitsgang (ein Gefäss) ohne Isolierung des Zwischenderivats der Formel VI erfolgt.

4. Verfahren nach Anspruch 3, dadurch gekennzeichnet, dass die Reaktion bei einer zwischen −78°C und +150°C liegenden Temperatur durchgeführt wird.

5. Verfahren nach Anspruch 1, dadurch gekennzeichnet, dass die Base $B^{\ominus}$ $M^{\oplus}$ ein Hydrid eines Alkalimetalls, eine Alkalimetallalkylverbindung oder ein Alkali- oder Erdalkalimetallalkoholat ist.

6. Verfahren nach Anspruch 5, dadurch gekennzeichnet, dass $M^{\oplus}$ ein aus Natrium, Lithium, Kalium und Magnesium ausgewähltes Metall ist.

7. Verfahren nach Anspruch 1, dadurch gekennzeichnet, dass die Base $B'^{\ominus}$ $M'^{\oplus}$ analog der in Anspruch 6 definierten Base $B^{\ominus}$ $M^{\oplus}$ ist.

8. Verfahren nach Anspruch 1, dadurch gekennzeichnet, dass die Reduktion des Derivats der Formel IX mit einem gemischten Alkalimetallhydrid, insbesondere Natriumborhydrid oder Kaliumborhydrid durchgeführt wird.

**Claims for the Contracting States:**
**BE, CH, DE, FR, GB, IT, LI, LU, NL, SE**

1. Process for the preparation of 2-(2-thienyl)- and -(3-thienyl)ethylamine derivatives of the formula:

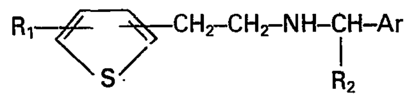

(I)

in which:

$R_1$ at 2-, 3-, 4-, or 5-position represents a hydrogen atom, a halogen atom, a nitro, amino, cyano, carboxyl or straight- or branched-chain alkyl or alkoxy radical;

$R_2$ represents a hydrogen atom or a straight- or branched-chain alkyl radical;

Ar represents a thienyl, furyl, pyridyl, phenyl or naphtyl radical, or a phenyl optionally mono- or polysubstituted with a halo, nitro, cyano, amino, carboxy, alkyl, alkoxy or phenyl group;

comprising condensing a derivative of the formula:

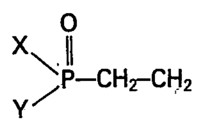

(II)

in which X and Y represent independently from each other an alkyl, alkoxy, aryl, aryloxy, diaryl- or dialkylamino radical, with a carbonyl compound of the formula:

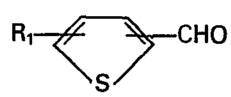

(III)

in which $R_1$ has the above-defined meaning, to give a compound of the formula (IV):

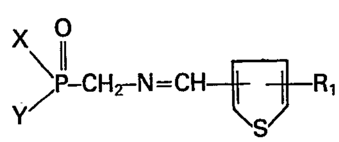

(IV)

in which X, Y and $R_1$ have the above defined meanings;

– treating compound (IV) with a base $B^{\ominus}$ $M^{\oplus}$, to give a carbanion of the formula (V):

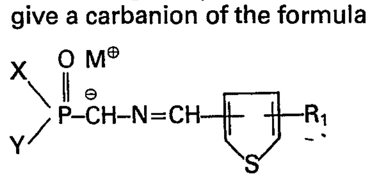

(V)

in which X, Y and $R_1$ have the above defined meanings;

– converting compound (V) to a derivative of the formula (VI):

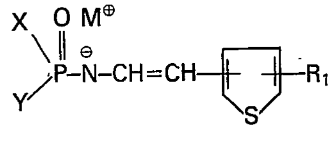

(VI)

under the action of heat, to give, after taking up into water, a derivative of the formula (VII):

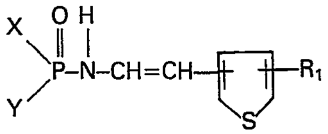

(VII)

– treating compound (VII) sequentially with a base $B'^{\ominus}$ $M'^{\oplus}$ and with a carbonyl compound of the formula (VIII)

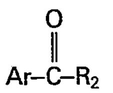

(VIII)

in which Ar and $R_2$ are as previously defined, to give a derivative of the formula (IX):

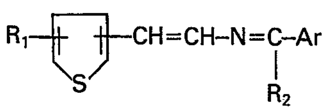

(IX)

and treating compound (IX) with a reducing agent, to give a compound of the formula (I).

2. Process as claimed in claim 1, wherein the reaction between organophosphorus compound (II) and carbonyl compound (III) is catalyzed by the presence of an inorganic or organic acid such as paratoluenesulfonic acid.

3. Process as claimed in claim 1, wherein the conversion of the carbanion of the formula (V) to a derivative of the formula (VII) is effected in one pot, without isolation of intermediate derivative of the formula (VI).

4. Process as claimed in claim 3, wherein the reaction is effected at a temperature comprised between −78°C and +150°C.

5. Process as claimed in claim 1, wherein base $B^{\ominus}$ $M^{\oplus}$ is an alkali metal hydride, an alkyl-alkali metal compound or an alkali or alkaline-earth metal alcoholate.

6. Process as claimed in claim 5, wherein $M^{\oplus}$ is a metal selected from sodium, lithium, potassium and magnesium.

7. Process as claimed in claim 1, wherein the base $B'^{\ominus}$ $M'^{\oplus}$ is analogous to the base $B^{\ominus}$ $M^{\oplus}$ defined in claims 5 or 6.

8. Process as claimed in claim 1, wherein the reduction of the derivative of the formula (IX) is effected with a mixed alkali metal hydride, typically sodium borohydride or potassium borohydride.

9. N-orthochlorobenzyl 2-[5-terbutoxy (2-thienyl)] ethylamine.

10. N-2-furfuryl 2-(2-thienyl) ethylamine.

11. N-orthonitrobenzyl 2-(2-thienyl) ethylamine.

12. N-(4-picolyl) 2-(2-thienyl) ethylamine.

13. N-(2-thenyl) 2-(2-thienyl) ethylamine.

14. N-orthochlorobenzyl 2-(3-thienyl) ethylamine.

**Claims for the Contracting State: AT**

1. Process for the preparation of 2-(2-thienyl)- and -(3-thienyl)ethylamine derivatives of the formula:

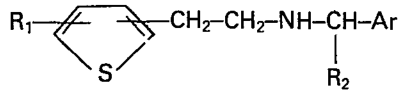

(I)

in which:

$R_1$ at 2-, 3-, 4-, or 5-position represents a hydrogen atom, a halogen atom, a nitro, amino, cyano, carboxyl or straight- or branched-chain alkyl or alkoxy radical;

$R_2$ represents a hydrogen atom or a straight- or branched-chain alkyl radical;

Ar represents a thienyl, furyl, pyridyl, phenyl or naphtyl radical, or a phenyl optionally mono- or polysubstituted with a halo, nitro, cyano, amino, carboxy, alkyl, alkoxy or phenyl group;

comprising condensing a derivative of the formula:

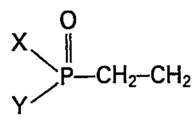

(II)

in which X and Y represent independently from each other an alkyl, alkoxy, aryl, aryloxy, diaryl- or dialkylamino radical, with a carbonyl compound of the formula:

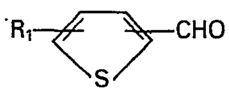

(III)

in which $R_1$ has the above-defined meaning, to give a compound of the formula (IV):

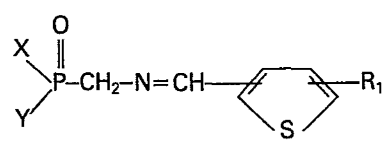

(IV)

in which X, Y and $R_1$ have the above defined meanings;

– treating compound (IV) with a base $B^{\ominus}$ $M^{\oplus}$, to give a carbanion of the formula (V):

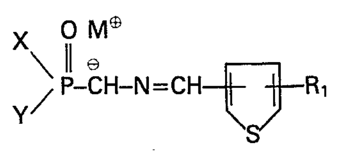

(V)

in which X, Y and $R_1$ have the above defined meanings;

– converting compound (V) to a derivative of the formula (VI):

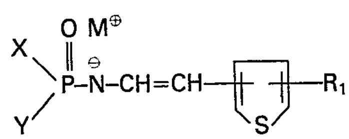

(VI)

under the action of heat, to give, after taking up into water, a derivative of the formula (VII):

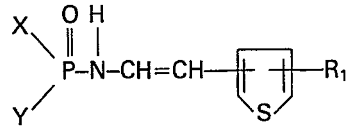

(VII)

– treating compound (VII) sequentially with a base $B'^{\ominus}$ $M'^{\oplus}$ and with a carbonyl compound of the formula (VIII)

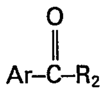
(VIII)

in which Ar and $R_2$ are as previously defined, to give a derivative of the formula (IX):

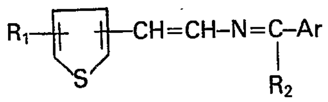
(IX)

and treating compound (IX) with a reducing agent, to give a compound of the formula (I).

2. Process as claimed in claim 1, wherein the reaction between organophosphorus compound (II) and carbonyl compound (III) is catalyzed by the presence of an inorganic or organic acid such as paratoluenesulfonic acid.

3. Process as claimed in claim 1, wherein the conversion of the carbanion of the formula (V) to a derivative of the formula (VII) is effected in one pot, without isolation of intermediate derivative of the formula (VI).

4. Process as claimed in claim 3, wherein the reaction is effected at a temperature comprised between −78°C and +150°C.

5. Process as claimed in claim 1, wherein base $B^{\ominus}$ $M^{\oplus}$ is an alkali metal hydride, an alkyl-alkali metal compound or an alkali or alkaline-earth metal alcoholate.

6. Process as claimed in claim 5, wherein $M^{\oplus}$ is a metal selected from sodium, lithium, potassium and magnesium.

7. Process as claimed in claim 1, wherein the base $B'^{\ominus}$ $M'^{\oplus}$ is analogous to the base $B^{\ominus}$ $M^{\oplus}$ defined in claims 5 or 6.

8. Process as claimed in claim 1, wherein the reduction of the derivative of the formula (IX) is effected with a mixed alkali metal hydride, typically sodium borohydride or potassium borohydride.